# EUROPEAN PATENT SPECIFICATION

(11) **EP 2 349 259 B1**
(45) Date of publication and mention of the grant of the patent: **23.12.2015**
(21) Application number: 09748884.5
(22) Date of filing: 16.10.2009
(51) Int. Cl.: A61K 31/433, A61P 35/00

(54) **INHIBITORS OF MITOSIS FOR INCREASING APOPTOSIS IN THERAPY**
MITOSE-HEMMER ZUR VERSTÄRKUNG DER APOPTOSE IN DER THERAPIE
INHIBITEURS DE LA MITOSE PERMETTANT D AUGMENTER L APOPTOSE EN THÉRAPIE

(30) Priority: 16.10.2008 US 106086 P
(43) Date of publication of application: 03.08.2011
(73) Proprietor: Array Biopharma, Inc., Boulder, CO 80301 (US)
(72) Inventor: TUNQUIST, Brian, J., Boulder, Colorado 80301 (US); WALKER, Duncan, H., Boulder, Colorado 80301 (US); WOESSNER, Richard, Donald, Boulder, Colorado 80301 (US)
(74) Representative: Office Freylinger
(86) International application number: PCT/US2009/061106
(87) International publication number: WO 2010/045624

(56) References cited:
- WO-A2-2006/044825
- WOESSNER R ET AL: "447 POSTER ARRY-520, a KSP inhibitor with potent in vitro and in vivo efficacy and pharmacodynamic activity in models of multiple myeloma" EUROPEAN JOURNAL OF CANCER. SUPPLEMENT, PERGAMON, OXFORD, GB, vol. 6, no. 12, 1 October 2008 (2008-10-01), pages 140-141, XP025534511 ISSN: 1359-6349 [retrieved on 2008-10-01]

## Description

### FIELD OF THE INVENTION

The present invention relates to an inhibitor of mitosis for administration to a patient having pathogenic cells in a state of mitotic inhibitor-induced mitotic arrest, for increasing apoptosis of the cells.

### DESCRIPTION OF THE STATE OF ART

Inhibitors of mitosis (also called mitotic inhibitors or anti-mitotics) are important therapeutics for the treatment of diseases, and they are used in treatments for cancer, as well as anti-gout and anti-fungus agents and treating restenosis. These inhibitors of mitosis therapeutics disrupt mitosis such that the cell will no longer divide. In cancer, inhibitors of mitosis can stop cancerous growth and lead to apoptosis or exit from mitosis followed by cell death.

Many inhibitors of mitosis are known. Some inhibitors of mitosis are anti-tubulin agents. Anti-tubulin agents act on tubulin, a protein that is necessary for mitosis. Anti-tubulin agents include vinca alkaloids, taxanes and epothilones. Non-tubulin targeted inhibitors of mitosis have also been investigated as cancer therapeutics. Different inhibitors of mitosis affect different portions of the cell cycle, and sometimes other functions outside of mitosis. For instance, anti-tubulin agents can affect non-mitotic cytoskeletal functions in proliferating cells and in terminally differentiated cells. Peripheral neurotoxicity has been associated with tubulin agents. Thus, different inhibitors of mitosis may have different toxicities.

Vinca alkaloids inhibit microtubule polymerization, which thereby inhibits mitosis. Vinca alkaloids include vinblastine, vincristine, vindesine and vinorelbine. Vinblastine has been used to treat certain kinds of cancer, including Hodgkin's lymphoma, non-small cell lung cancer, breast cancer and testicular cancer. Vincristine has been used to treat certain kinds of cancer, including lymphoma, breast cancer, lung cancer and acute lymphoblastic leukemia. Vinblastine and vincristine have also been used in palliative regimens for some of the major solid tumors (See Wood, Kenneth W., et al. "Past and future of the mitotic spindle as an oncology treatment." Current Opinion in Pharmacology. Vol. 1, Issue 4 (August 1, 2001): pp. 370-377). Vindesine has been used to treat certain kinds of cancer, including leukemia, lymphoma, melanoma, breast cancer and lung cancer. Vinorelbine has been used to treat certain kinds of cancer, including breast cancer and non-small cell lung cancer.

Taxanes stabilize microtubules, thereby inactivating the microtubule function of a cell and inhibiting cell division. Taxanes include paclitaxel (including Abraxane*) and docetaxel. Paclitaxel is used to treat certain kinds of cancer, including lung cancer, ovarian cancer, breast cancer and advanced forms of Kaposi's sarcoma. Docetaxel is used to treat certain kinds of cancer, including breast cancer, ovarian cancer and non-small cell lung cancer. New taxanes are also in development, for example BMS275183 (See 2006 EJC Poster: Broker, L.E., et al "The novel oral taxanes BMS275183 has a favorable activity and toxicity profile in a twice weekly schedule; Preliminary findings from an extended phase 1 trial." EJC Suppl. 2006 Abstract 644, p. 194).

Additionally, colchicine is an inhibitor of mitosis that acts as an anti-tubulin agent. Colchicine inhibits mitosis by inhibiting microtubule polymerization. Colchicine is used to treat gout.

Epothilones are a class of microtubule-stabilizing chemotherapeutic agents with activity in paclitaxel-resistant cancer cell lines (See Denduluri, Neelima, et al. "Phase II trial of ixabepilone, an epothilones B analog, given daily for three days every three weeks, in metastatic breast cancer." Invest. New Drugs. 25 (August 25, 2006): pp. 63-67). Epothilones include epothilone A, epothilone B, epothilone D and the epothilone analog ixabepilone. Ixabepilone has been approved for the treatment of aggressive metastatic or locally advanced breast cancer no longer responding to currently available chemotherapies.

Dolastatin and dolastatin analogues are inhibitors of mitosis. These compounds include dolastatin 10, dolastatin 15, synthadotin (or SYN-D or ILX651; see 2004 ASCO Abstract No. 3068, Hammond, L.A., et al. "Phase (Ph) 1 evaluation of the dolastatin analogue synthadotin (SYN-D; ILX651): Pooled data analysis of three alternate schedules in patients (pts) with advanced solid tumors." J. Clin. Oncology. 2004 Suppl. Abstract 3068 14s (2004)), LU103793 and cemadotin.

Aurora kinases, including Aurora A, Aurora B and Aurora C, are serine/threonine kinases that function in mitosis. Aurora kinases have been targeted as inhibitors of mitosis. Aurora A has its function in the prophase of mitosis and is required for the centrosomes to function correctly. Aurora B functions in the attachment of the mitotic spindle to the centromere. Aurora kinase inhibitors include AZD-1152, CYC-116, AS-703569 (or R-763). MLN-8054, PHA-739358, AT-9283, SNS-314, AZD-1152-HQPA, MLN-8237, KW-2449, PF-3814735, ENMD-2076 (or ENMD-981693), PHA-739385, MK-0457 (or VX-680) and MK-5108 (or VX-689). For more, see: Gautschi, Oliver, et al. "Aurora Kinases as Anticancer Drug Targets." Clin. Cancer Res. 14(6) (March 15, 2008): pp. 1639-48.

Polo-like kinases ("Plks"), including polo-like kinase 1 ("Plk1"), polo-like kinase 2 ("Plk2"), polo-like kinase 3 ("Plk3") and polo-like kinase 4 ("Plk4"), are involved in the formation and changes in the mitotic spindle and in the activation of CDK/cyclin complexes during mitosis. Polo-like kinases have been targeted as inhibitors of mitosis. Polo-like kinase inhibitors include ON-01910Na (or ON-1910Na or Onc-01910), BI-2536 (See: Steegmaier, Martin, et al. "B1 2536, a Potent and Selective Inhibitor of Polo-like Kinase 1, Inhibits Tumor Growth In Vivo." Current Biology, 17 (February 20, 2007): pp. 316-322) and GSK-61364 (or GSK-461364A).

Kinesins are a type of motor protein. Mitotic kinesins are enzymes essential for assembly and function of the mitotic spindle. Mitotic kinesins play essential roles during all phases of mitosis. During mitosis, kinesins organize the microtubules into the bipolar structure that is the mitotic spindle. Inhibition of mitotic kinesin causes malformation or dysfunction of the mitotic spindle, frequently resulting in cell cycle arrest and apoptosis (cell death).

Among the identified mitotic kinesins is kinesin spindle protein ("KSP"). During mitosis, KSP associates with microtubules of the mitotic spindle. Inhibition of KSP prevents spindle pole separation during the prometaphase, giving rise to monopolar spindles causing mitotic arrest and induction of programmed cell death. Human KSP is also called HsEg5.

United States Patent Application Publication 2006/0100161 describes compounds including 2-(3-aminopropyl)-5-(3-fluorophenyl)-N-(2-methoxyethyl)-N-methyl-2-phenyl-1,3,4-thiadiazole-3(2H)-carboxamide (hereinafter "Compound 1"). 2-(3-aminopropyl)-5-(3-fluorophenyl)-N-methoxy-N-methyl-2-phenyl-1,3,4-thiadiazole-3(2H)-carboxamide (hereinafter "Compound 2"), 2-(3-aminopropyl)-5-(2,5-difluorophenyl)-N-methoxy-N-methyl-2-phenyl-1,3,4-thiadiazole-3(2H)-carboxamide (hereinafter "Compound 3"), (S)-2-(3-aminopropyl)-5-(2,5-difluorophenyl)-N-methoxy-N-methyl-2-phenyl-1,3,4-thiadiazole-3(2H)-carboxamide (hereinafter "Compound 4"), (R)-2-(3-aminopropyl)-5-(2.5-difluorophenyl)-N-methoxy-N-methyl-2-phenyl-1,3,4-thiadiazole-3(2H)-carboxamide (hereinafter "Compound 5"), and 2-(3-aminopropyl)-5-(2,5-difluorophenyl)-N-hydroxy-N-methyl-2-phenyl-1,3,4-thiadiazole-3(2H)-carboxamide (hereinafter "Compound 6"). Compounds 1, 2, 3, 4, 5 and 6 (collectively the "'161 KSP Inhibitors") are KSP inhibitors.

KSP inhibitors include ispinesib (or SB-715992 or CK-0238273; See 2008 ASCO Poster: "A Phase I-II Open-Label Trial of *Ispinesib* on an Alternating Dosing Schedule in Chemotherapy-Naive Patients with Locally Advanced or Metastatic Breast Cancer (MBC)." www.cytokinetics.com/pdf/ASCO2008A.pdf), the '161 KSP Inhibitors, AZD-4877, CRx-026. SB-743921 (SB-921), MK-0731, EMD-534085 and ARQ 621. Ispinesib has been tested in a wide range of tumor types and is being tested in human clinical trials.

Among the other motor proteins that act during mitosis, small molecule inhibitors have also been described for centromere associated protein E ("CENP-E"). CENP-E is a motor protein (See Chan, G.K.T., et al. "Characterization of the Kinetochore Binding Domain of CENP-E Reveals Interactions with the Kinetochore Proteins CENP-F and hBUBRI." J. Cell Biology. Vol. 143, No. I (October 5, 1998): pp. 49-63) and can be classified as a type of mitotic kinesin. CENP-E inhibitors include GSK-295 (or GSK-923295).

Many inhibitors of mitosis have been tested as therapeutics for the treatment of diseases. Some inhibitors of mitosis have been administered on a one day schedule, either weekly, biweekly, monthly, and including 24-hour infusions. By administering only one dose, the inhibitors of mitosis may not keep the cells in mitotic arrest long enough for the cells to go to apoptosis or exit mitosis and go to cell death. Also, some inhibitors of mitosis have been administered twice a week, three times a week or three times a month. Administering several doses over longer period of time often decreases the dose patients are able to tolerate, and the individual doses may not reach a biologically effective level.

### SUMMARY OF THE INVENTION

Surprisingly, it has been found that after a first dose of an inhibitor of mitosis has been administered to a mammal with pathogenic cells, and the cells have entered into mitotic arrest, administration of a second dose of the inhibitor of mitosis one or two days after the first dose increases apoptosis or exit from mitosis followed by cell death.

In one aspect, the present invention relates to an inhibitor of mitosis for administration to a patient having pathogenic cells in a state of mitosis inhibitor-induced mitotic arrest, for increasing apoptosis of the cells.

Another aspect of the present invention provides the '161 KSP Inhibitors for administration to a patient having pathogenic cells in a state of '161 KSP Inhibitor induced mitotic arrest, for increasing apoptosis of the cells.

### BRIEF DESCRIPTION OF THE FIGURES

Figure 1 shows an apoptosis washout experiment.
Figure 2 shows caspase 3/7 activity over time in HT-29 cells in vitro.
Figure 3 shows caspase 3/7 activity over time in RPMI 8226 cells in vitro.
Figure 4 shows the amount of monopolar spindles in subcutaneous HT-29 xenografts in nude mice at various time points for two different dosing schedules.
Figure 5 shows the amount of monopolar spindles in subcutaneous HT-29 xenografts in nude mice at various time points for two different dosing schedules.
Figure 6 shows the percentage of apoptotic cells in subcutaneous HT-29 xenografts in nude mice at various time points for two different dosing schedules.
Figure 7 shows the percentage of apoptotic cells in subcutaneous HT-29 xenografts in nude mice at various time points for two different dosing schedules.
Figure 8 shows the percentage of cells with monopolar spindles and bipolar spindles in subcutaneous HT-29 xenografts in nude mice at 24 hours and 48 hours for various dose amounts.
Figure 9 shows the percentage of apoptotic cells in subcutaneous HT-29 xenografts in nude mice at 24 hours and 48 hours for various dose amounts.
Figure 10 shows a tumor growth inhibition ("TGI") experiment in nude mice with subcutaneous HT-29 xenografts.
Figure 11 shows a TGI experiment in nude mice with subcutaneous HT-29 xenografts.
Figure 12 shows a TGI experiment in nude mice with subcutaneous HT-29 xenografts.
Figure 13 shows a TGI experiment in nude mice with subcutaneous HT-29 xenografts.
Figure 14 shows a TGI experiment in nude mice with subcutaneous HT-29 xenografts.
Figure 15 shows a TGI experiment in nude mice with subcutaneous HT-29 xenografts.
Figure 16 shows a TGI experiment in nude mice with subcutaneous HT-29 xenografts.
Figure 17 shows the percentage of monopolar spindles in subcutaneous RPMI8226 xenografts in SCID-beige mice at various time points for different dosing schedules.
Figure 18 shows the percentage of apoptotic cells in subcutaneous RPMI 8226 xenografts in SCID-beige mice at various time points after for different dosing schedules.
Figure 19 shows the percentage of bipolar spindles in subcutaneous RPMI 8226 xenografts in SCID-beige mice at various time points for different dosing schedules.

### DETAILED DESCRIPTION OF THE INVENTION

Reference will now be made in detail to certain embodiments of the invention. While the invention will be described in conjunction with the enumerated embodiments, it will be understood that they are not intended to limit the invention to those embodiments. On the contrary, the invention is intended to cover all alternatives, modifications, and equivalents, which may be included within the scope of the present invention as defined by the claims. One skilled in the art will recognize many methods and materials similar or equivalent to those described herein, which could be used in the practice of the present invention. The present invention is in no way limited to the methods and materials described. In the event that one or more of the incorporated literature and similar materials differs from or contradicts this application, including but not limited to defined terms, term usage, described techniques, or the like, this application controls.

### DEFINITIONS

The terms "cancer" and "cancerous" refer to or describe the physiological condition in mammals-that is typically characterized by unregulated cell growth. A "tumor" comprises one or more cancerous cells. Examples of cancer include, but are not limited to, carcinoma, lymphoma, blastoma, sarcoma, and leukemia or lymphoid malignancies. More particular examples of such cancers include squamous cell cancer (e.g., epithelial squamous cell cancer), lung cancer including small cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, skin cancer including melanoma, head and neck cancer, multiple myeloma and acute myeloid leukemia.

The terms "treat" or "treatment" refer to therapeutic, prophylactic, palliative or preventative measures. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder, as well as those prone to have the condition or disorder or those in which the condition or disorder is to be prevented.

### INHIBITORS OF MITOSIS FOR INCREASING APOPTOSIS IN THERAPY

The present invention provides an inhibitor of mitosis for administration to a patient having pathogenic cells in a state of mitosis inhibitor-induced mitotic arrest, for increasing apoptosis of the cells.

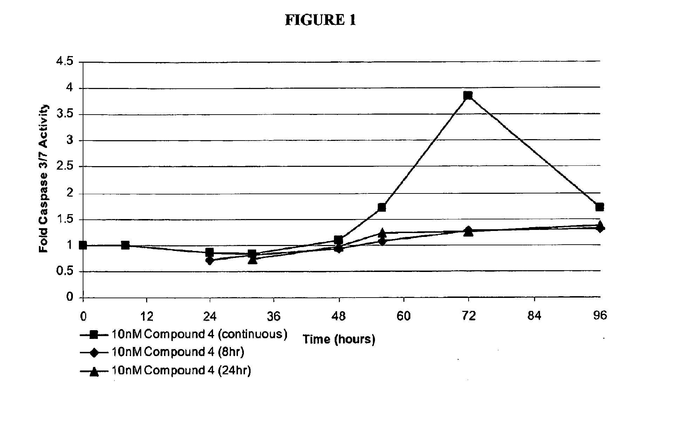

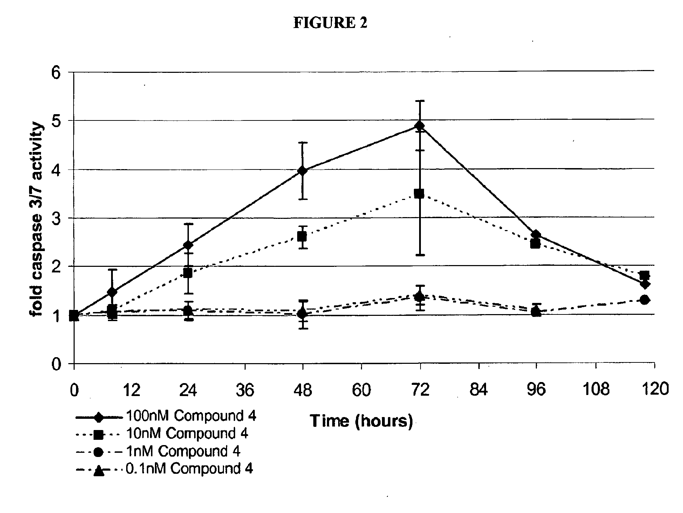

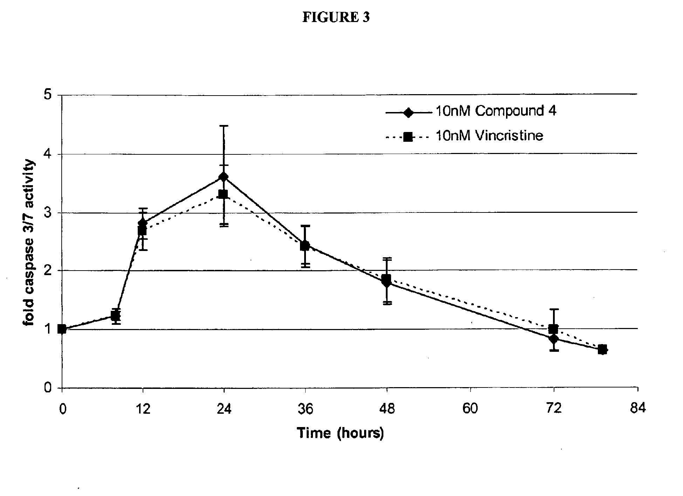

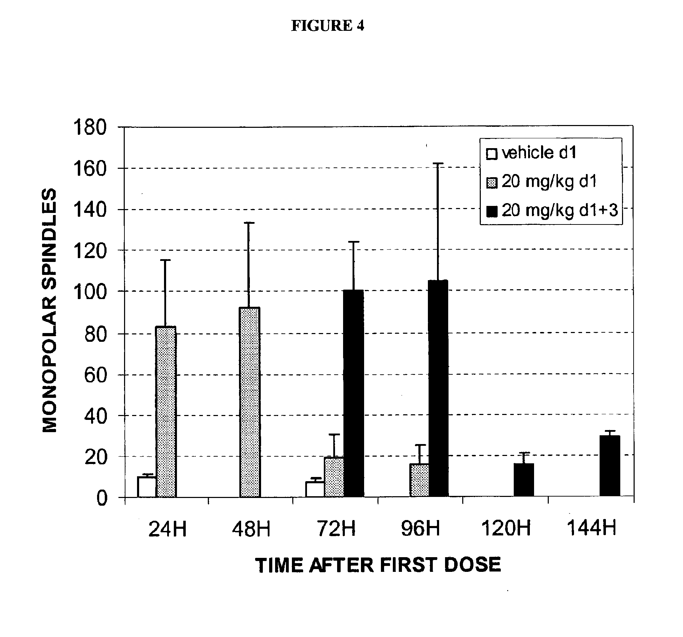

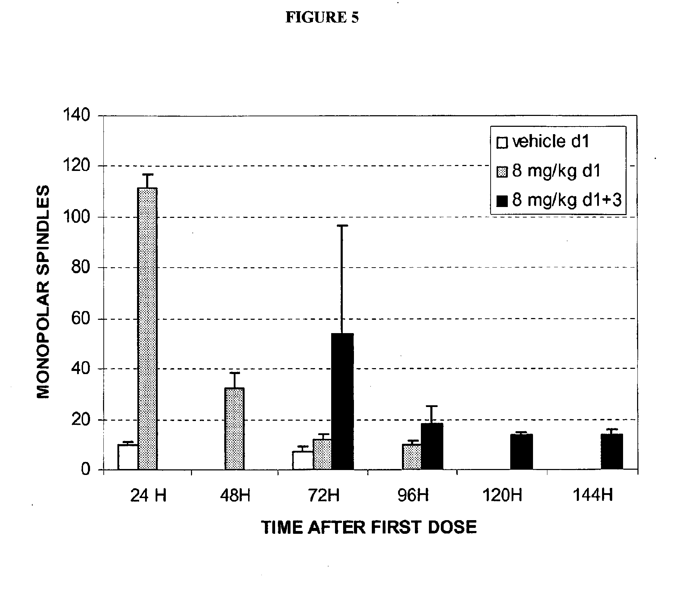

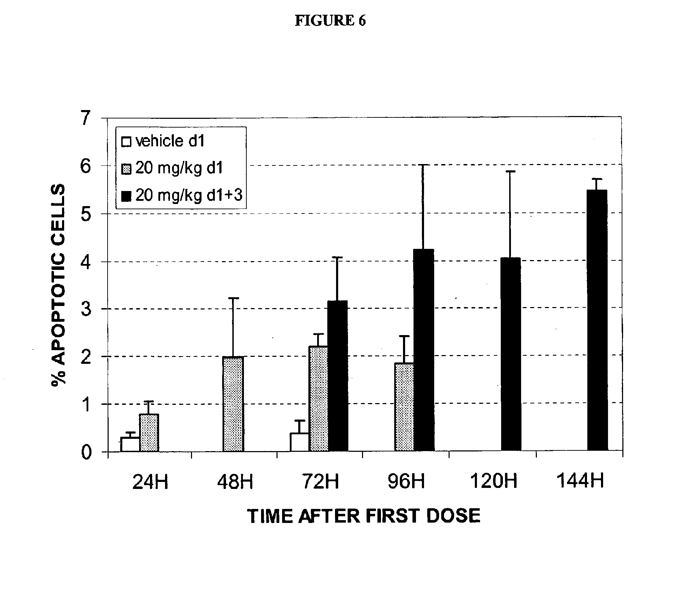

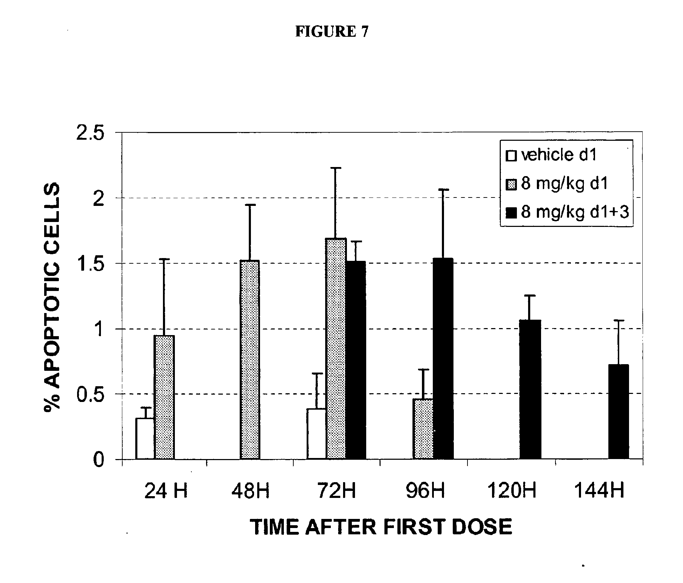

Administering an inhibitor of mitosis to cells puts the cells into mitotic arrest. However, mitotic arrest does not necessarily lead the cells to apoptosis or result in antitumor efficacy (See, for example: Shi, Jue, et al. "Cell Type Variation in Responses to Antimitotic Drugs that Target Microtubules and Kinesin-5." Cancer Research. 68(9) (May 1, 2008): pp. 3269-76; and 2002 AACR Poster: "A Pharmacodynamic marker of mitosis demonstrates the anti-mitotic activity of SB-715992, an inhibitor of the mitotic kincsin KSP." www.cytokinetics.com/pdf/AACR_2002_Poster_1336.pdf). It has been found that the cells must stay in arrest for a duration of time before apoptosis peaks (See Figure 1) The duration of time needed for apoptosis is variable between cell types and types of tumors (See Figures 2 and 3). Also, administering two doses instead of one dose increases the duration of the biological effect (See Figures 4 and 5), which in the case of inhibitors of mitosis increases the duration and magnitude of apoptosis (See Figures 6 and 7). Therefore, keeping the cells in arrest for an appropriate duration of time to be effective is necessary to increase apoptosis using an inhibitor of mitosis.

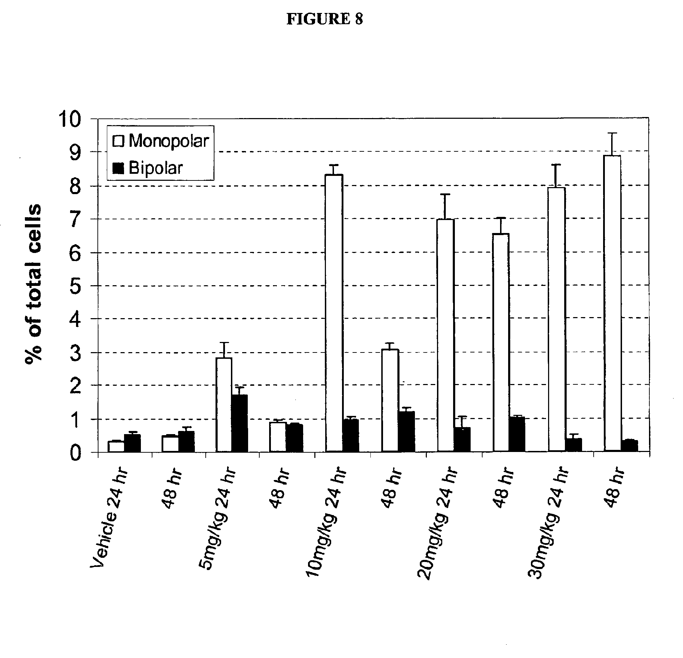

Administering an inhibitor of mitosis to cells interferes with mitosis. For example, administering a KSP inhibitor increases the amount of monopolar spindles. However, a minimum amount of the inhibitor must be administered in order to achieve the desired biological response (See Figure 8). Therefore, administration of an inhibitor of mitosis must achieve a biologically effective dose of the inhibitor to be effective. The biologically effective dose of a KSP inhibitor is the dose of the inhibitor that results in the appearance of arrested, monopolar spindles. These can be seen by immunohistochemical techniques (See Figures 4, 5 and 8). The biologically effective dose of other inhibitors of mitosis will result in mitotic aberrations consistent with their target profile.

If the administration of the inhibitor of mitosis fails to reach the biologically effective dose, then the proper biological response will not transpire. Also, if the administration of the inhibitor fails to hold the cells in arrest long enough, the cells may not go to apoptosis. Therefore, effectively increasing apoptosis using an inhibitor of mitosis requires the inhibitor of mitosis to be administered at least at the biologically effective dose to get the intended biological effect (i.e., mitotic arrest), as well as, being dosed for a period of time long enough to keep the cells in arrest and induce apoptosis (See Figures 4-9 and 17-19).

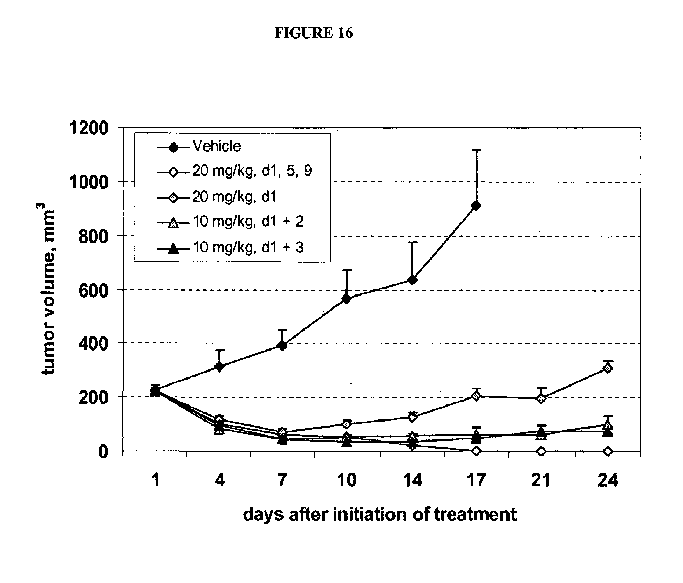

It has been found that administering an inhibitor of mitosis as a split dose divided over two days may be more efficacious than the same total dose given on one day (See Figure 16).

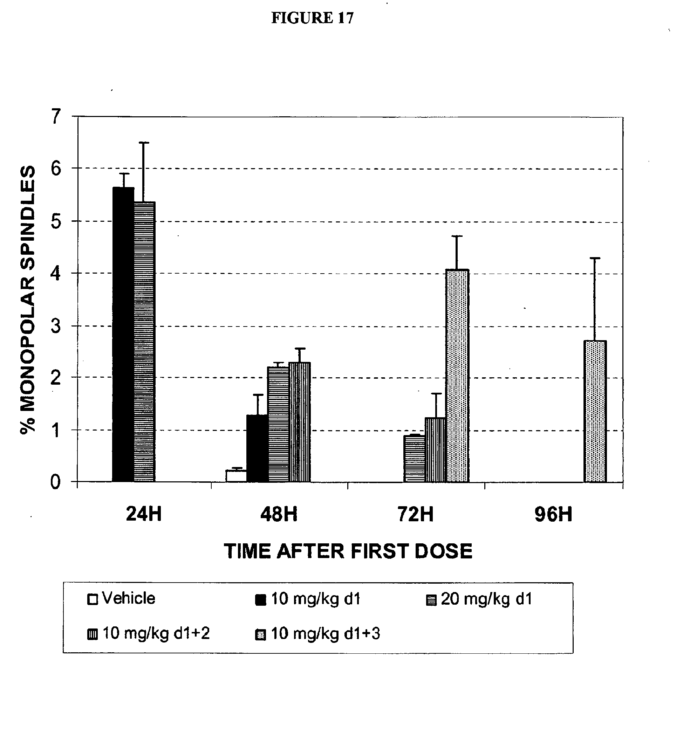

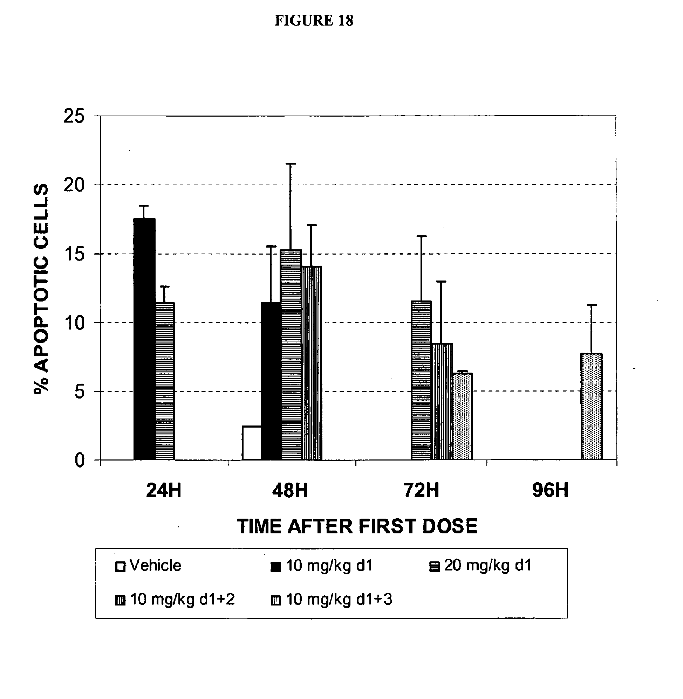

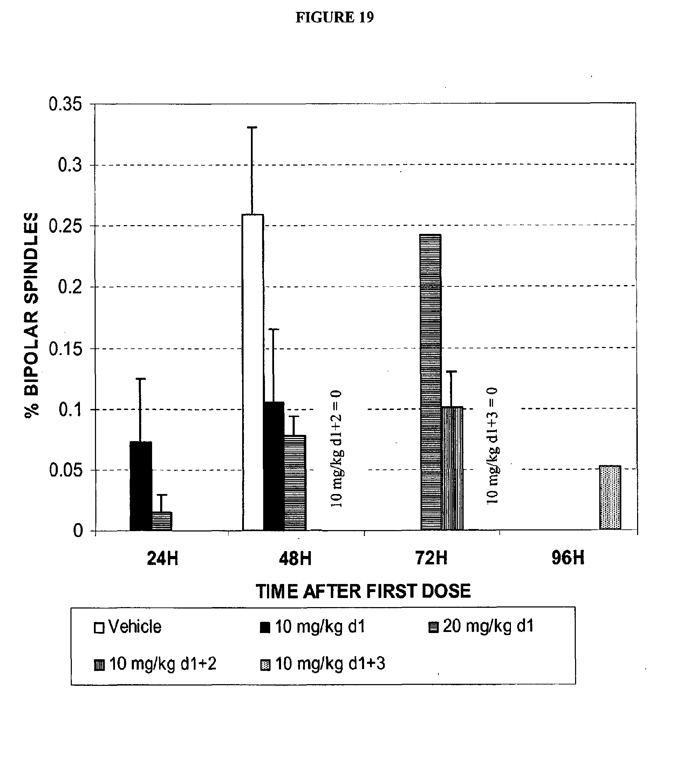

For tumors in which cells rapidly enter apoptosis following mitotic block (See Figure 3), mitotic arrest (See Figure 17) or apoptosis (See Figure 18) may not directly correlate with the enhanced efficacy for inhibiting tumor growth on a divided dose schedule (See Figure 16). In such cases, the fewer cells observed in mitotic arrest and apoptosis may reflect the rapid cell death, such that they are no longer detectable in the tumor. However, the amount of cells with bipolar spindles, indicative of normally cycling cells in mitosis, may inversely correlate with enhanced efficacy (See Figure 19). In such cases, fewer cells with bipolar spindles indicate a more complete mitotic block, with fewer cells escaping the block and re-entering the cell cycle.

One embodiment of the present invention provides an inhibitor of mitosis for administration to a patient having pathogenic cells in a state of mitosis inhibitor-induced mitotic arrest, for increasing apoptosis of the cells.

Another embodiment of the present invention provides a '161 KSP Inhibitor for administration to a patient having pathogenic cells in a state of '161 KSP Inhibitor-induced mitotic arrest, for increasing apoptosis of the cells.

The present invention is directed to administering the same inhibitor of mitosis to increase apoptosis as the inhibitor of mitosis administered to induce mitotic arrest.

The present invention is useful for treating pathogenic cells caused by cell division or that are treated by inhibiting mitosis. Inhibitors of mitosis can be used to treat a variety of diseases, including hyperproliferative diseases and gout. Hyperproliferative diseases include cancer, autoimmune disease, arthritis, graft rejection, inflammatory bowel disease, or proliferation induced after a medical procedure.

In certain embodiments, the invention provides increased apoptosis for pathogenic cancer cells. More particularly, the pathogenic cancer cells include, but are not limited to: Soft Tissue Cancers: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucasonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulose-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic: blood and bone marrow (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; Skin: malignant melanoma, basal cell carcinoma, squamous cell carcinoma, Kaposi's sarcoma, moles dysplastic nevi, lipoma, angioma, dennatonbroma, keloids, psoriasis; and Adrenal glands: neuroblastoma. The term "cancerous cell" as provided herein, includes a cell afflicted by any one of the above identified conditions.

In certain embodiments, the present invention is useful for increasing apoptosis of pathogenic cancer cells, wherein the pathogenic cancer cells are solid tumor cells. The solid tumor cells include tumor cells of the skin, breast, brain, cervical carcinoma, testicular carcinoma cells, etc. In certain embodiments, the solid tumors are selected from breast cancer, colorectal cancer, non-small cell lung cancer, pancreatic cancer, bladder cancer, salivary gland cancer (adenoid cystic), esophageal cancer, mesothelioma cancer, and mixed small cell lung cancer / non-small cell lung cancer.

In certain embodiments, the present invention is useful for increasing apoptosis of pathogenic cancer cells, wherein the pathogenic cancer cells are hematological tumor cells. The hematological tumor cells include lymphomas, leukemia, multiple myeloma cells and the like. In certain embodiments, the present invention is useful for increasing apoptosis of pathogenic cancer cells, wherein the pathogenic cancer cells are selected from lymphomas, leukemia and multiple myeloma cells. In a further embodiment, the present invention is useful for increasing apoptosis of pathogenic cancer cells, wherein the pathogenic cancer cells are advanced myeloid leukemia, or relapsed or refractory multiple myeloma cells. In a further embodiment, the present invention is useful for increasing apoptosis of pathogenic cancer cells, wherein the pathogenic cancer cells are relapsed or refractory multiple myeloma cells.

There are a multitude of variables when looking for increased apoptosis in administering inhibitors of mitosis. Particularly with inhibitors of mitosis, administration needs to continue for a period of time long enough and at a sufficient exposure level to be biologically effective.

In order to induce mitotic arrest in pathogenic cells, a first administration of an inhibitor of mitosis is administered. This first administration is said to be on day one. Then increased apoptosis may occur when a second administration of the inhibitor of mitosis is provided on day two or day three. Alternatively, the second dose is within 24 to 48 hours of the first dose. This aspect of the present method allows for increased apoptosis of the pathogenic cells because the inhibitor is being dosed high enough to achieve the biologically effective dose to get the intended biological effect, i.e., the cells are held in mitotic arrest, as well as holding the mitotic arrest long enough to promote apoptosis or exit of mitosis, which leads to cell death.

The timing of this second dose need not be exactly 24 to 48 hours after the first dose. This is just a convenient way of saying the second dose should be administered one or two days after the first dose. Therefore, the second dose is administered approximately 24 to 48 hours after the first dose. This second dose may be administered 12 to 60 hours after the first dose.

When administering the second dose of the inhibitor of mitosis, administering on back to back days allows for more convenience to the patients. It is preferable to have a convenient dosing schedule for patients to increase patient compliance with the method of treatment. This is especially true of therapeutics that are administered to patients via intravenous injection, as additional doses may require additional visits to a hospital or doctor to receive the injections.

Many types of inhibitors of mitosis are known, including vinca alkaloids, taxanes, epothilones, dolastatin and dolastatin analogues, aurora kinases, polo-like kinases, and mitotic kinesin inhibitors.

The inhibitor of mitosis may be selected from the group consisting of vinca alkaloids, taxanes, epothilones, dolastatin and dolastatin analogues, aurora kinase inhibitors, polo-like kinase inhibitors, and mitotic kinesin inhibitors.

The inhibitor of mitosis may be a mitotic kinesin inhibitor. The mitotic kinesin inhibitor may be a CENP-E inhibitor or a KSP inhibitor.

The inhibitor of mitosis may be a KSP inhibitor.

The inhibitor of mitosis may be selected from the group consisting of GSK-295, ispinesib, the '161 KSP Inhibitors, AZD-4877, CRx-026, SB-743921 (SB-921), MK-0731, EMD-534085 and ARQ 621.

The inhibitor of mitosis may be selected from the group consisting of ispinesib, the '161 KSP Inhibitors, AZD-4877, CRx-026, SB-743921 (SB-921), MK-0731, EMD-534085 and ARQ 621.

The inhibitor of mitosis may be selected from the group consisting of ispinesib, the '161 KSP Inhibitors and AZD-4877.

The inhibitor of mitosis may be selected from the group consisting of the '161 KSP Inhibitors. The inhibitor of mitosis may be Compound I. The inhibitor of mitosis may be Compound 2. The inhibitor of mitosis may be Compound 3. The inhibitor of mitosis may be Compound 4. The inhibitor of mitosis may be Compound 5. The inhibitor of mitosis may be Compound 6.

The inhibitor of mitosis may be selected from the group consisting of SU-6668, AZD-1152, CYC-116, AS-703569, MLN-8054, R763, PHA-739358, AT-9283. SNS-314, AZD-1152-HQPA, MLN-8237, KW-2449, PF-3814735, ENMD-2076, PHA-739385, MK-0457, MK-5108, ON-01910Na, BI-2536, GSK-461364, ispinesib, the '161 KSP Inhibitors. AZD-4877, CRx-026, SB-743921 (SB-921), MK-0731, EMD-534085, ARQ 621 and GSK-295.

The inhibitor of mitosis may be selected from the group consisting of vinblastine, vincristine, vindesine, vinorelbine, paclitaxel, docetaxel, Abraxane^{®}, colchicine, epothilone A, epothilone B, epothilone D, ixabepilone, dolastatin 10, dolastatin 15, synthadotin, LU103793, cemadotin, SU-6668, AZD-1152, CYC-116, AS-703569, MLN-8054, R763, PHA-739358, AT-9283, SNS-314, AZD-1152-HQPA, MLN-8237, KW-2449, PF-3814735, ENMD-2076, PHA-739385, MK-0457, MK-5108, ON-01910Na, BI-2336. GSK-461364, GSK-295, ispinesib, the '161 KSP Inhibitor. AZD-4877. CRx-026, SB-743921 (SB-921), MK-0731, EMD-534085, ARQ 621 and GSK-295.

The inhibitor of mitosis may be a vinca alkaloid. The vinca alkaloid may be selected from the group consisting of vinblastine, vincristine, vindesine and vinorelbine.

The inhibitor of mitosis may be a taxane. The inhibitor of mitosis may be selected from the group consisting of paclitaxel, Abraxane^{®} and docetaxel.

The inhibitor of mitosis may be colchicine.

The inhibitor of mitosis may be epothilone. The inhibitor of mitosis may be selected from the group consisting of epothilone A, epothilone B, epothilone D and ixabepilone.

The inhibitor of mitosis may be dolastatin or a dolastatin analogue. The inhibitor of mitosis may be selected from the group consisting of dolastatin 10, dolastatin 15, synthadotin, LU 103793 and cemadotin.

The inhibitor of mitosis may be an aurora kinase inhibitor. The inhibitor of mitosis may be selected from the group consisting of SU-6668, AZD-1152, CYC-116, AS-703569, MLN-8054, R763, PHA-739358, AT-9283, SNS-314, AZD-1152-HQPA, MLN-8237, KW-2449, PF-3814735, ENMD-2076. PHA-739385, MK-0457 and MK-5108.

The inhibitor of mitosis may be a polo-like kinase inhibitor. The inhibitor of mitosis may be selected from the group consisting of ON-01910Na, BI-2536 and GSK-461364.

The inhibitor of mitosis may be a CENP-E inhibitor. The inhibitor of mitosis may be GSK-295.

As discussed above, the proper amount of inhibitor of mitosis must be administered in order to reach the desired biological effect. Thus, to increase apoptosis by administering an inhibitor of mitosis will administer at least a minimum amount that reaches the desired biological effect, or biologically effective dose. However, the amount should not be so high as to outweigh the benefit of the biological effect with unacceptable side effects. Therefore, increasing apoptosis by administering an inhibitor of mitosis will administer no more than the maximum tolerated dose ("MTD"). Each administration of the inhibitor of mitosis is between the biologically effective dose and the maximum tolerated dose.

The maximum tolerated dose is defined as the highest dose that produces an acceptable incidence of dose-limiting toxicities ("DLT"). Doses that cause an unacceptable rate of DLT are considered non-tolerated. Typically, the MTD for a particular schedule is established in phase 1 clinical trials. These are usually conducted in patients by starting at a safe starting dose of 1/10 the severe toxic dose ("STD10") in rodents (on a mg/m² basis) and accruing patients in cohorts of three, escalating the dose according to a modified Fibonacci sequence in which ever higher escalation steps have ever decreasing relative increments (e.g., dose increases of 100%, 65%, 50%, 40%, and 30% to 35% thereafter). The dose escalation is continued in cohorts of three patients until a non-tolerated dose is reached. The next lower dose level that produces an acceptable rate of DLT is considered to be the MTD.

Also, the MTD of an inhibitor of mitosis varies depending on the inhibitor, species, formulation and dosing schedule. For instance, administering an inhibitor of mitosis only on day one versus days one and two versus days one through three over seven, fourteen, twenty-one or twenty-eight days may all have different MTDs. However, as discussed above, increasing apoptosis using an inhibitor of mitosis requires administering the inhibitor in a high enough amount to be biologically effective, as well as long enough to keep the cells in mitotic arrest. Administering on day one only may reach the biologically effective dose, but may not be long enough to increase apoptosis in the cells. Alternatively, administering the inhibitor of mitosis on days one through three may be long enough, but may not administer enough to reach the biologically effective dose, and thus apoptosis will not increase. This may be due to the MTD of dosing for three days being lower than the biologically effective dose.

Typically when treating pathogenic cells such as cancer, the MTD of a particular compound is administered to a patient so that the maximum benefit in the treatment can be reached. Accordingly, one embodiment of the present invention provides an inhibitor of mitosis for administration to a patient having pathogenic cells in a state of mitosis inhibitor-induced mitotic arrest, for increasing apoptosis of the cells, wherein the inhibitor of mitosis is administered at the maximum tolerated dose.

Another embodiment of the present invention provides the '161 KSP Inhibitors for administration to a patient having pathogenic cells in a state of '161 KSP Inhibitor-induced mitotic arrest, for increasing apoptosis of the cells, wherein the '161 Inhibitor is administered at the maximum tolerated dose.

When treating pathogenic cells such as cancer, a dosing cycle is established so that after the first cycle is completed, then additional cycles may be administered until such treatment is no longer necessary or effective. One of the factors in determining the length of a cycle is allowing for the recovery for subsiding of side effects. After administering a pharmaceutical composition or therapeutic, particularly an inhibitor of mitosis, patients may experience side effects. Depending on the type of side effects, a recovery or subsiding of side effects may be necessary. This recovery or subsiding of side effects may take time, which in turn can control the length of the cycle before a second cycle may begin.

One of the side effects of inhibitors of mitosis, and particularly KSP inhibitors, is acute neutropenia. Neutropenia is a hematological disorder characterized by an abnormally low number of neutrophil granulocytes, a type of white blood cell. Generally, patients who experience this type of side effect from an inhibitor of mitosis (or KSP inhibitor) recover or the neutropenia subsides as time passes without additional doses of the inhibitor.

After a single administering of a KSP inhibitor, many patients recover from the side effects or the side effects subside on day 14 to day 21 of the cycle.

The present invention provides an inhibitor of mitosis for administration to a patient having pathogenic cells in a state of mitosis inhibitor-induced mitotic arrest, for increasing apoptosis of the cells, wherein the cycle allows for the recovery or subsiding of side effects is achieved.

A first dose induces mitotic arrest. The present invention provides increased apoptosis with a second dose administered one or two days after the first dose. The present invention provides that the cycle including the first and second dose administration is 14 to 21 days. This is a convenient way of saying 2 or 3 weeks, and it does not necessarily need to be for exactly 14 to 21 days. Therefore, the cycle is approximately 14 to 21 days. The cycle may be from 11 to 24 days. The cycle may be 14 days, or 11 to 17 days. The cycle may also be 21 days, or 18 to 24 days.

Another embodiment of the present invention provides, a '161 KSP Inhibitor for administration to a patient having pathogenic cells in a state of '161 KSP Inhibitor-induced mitotic arrest, for increasing apoptosis of the cells. In certain embodiments, the '161 KSP Inhibitor is Compound 1. In certain embodiments, the'161 KSP Inhibitor is Compound 2. In certain embodiments, the '161 KSP Inhibitor is Compound 3. In certain embodiments, the '161 KSP Inhibitor is Compound 4. In certain embodiments, the '161 KSP Inhibitor is Compound 5. In certain embodiments, the '161 KSP Inhibitor is Compound 6. In certain embodiments, the pathogenic cells are cancer cells. In certain embodiments, the pathogenic cells are hematological tumor cells. In certain embodiments, the pathogenic cells are selected from lymphomas, leukemia and multiple myeloma cells. In certain embodiments, the pathogenic cells are solid tumor cells. In certain embodiments, the pathogenic ,cells are selected from tumor cells of the skin, breast, brain, cervical carcinoma, and testicular carcinoma cells. In certain embodiments, the solid tumor cells are selected from breast cancer, colorectal cancer, non-small cell lung cancer, pancreatic cancer, bladder cancer, salivary gland cancer (adenoid cystic), esophageal cancer, mesothelioma cancer, and mixed small cell lung cancer / non-small cell lung cancer. In certain embodiments, the Inhibitor is dosed at the maximum tolerated dose.

Another embodiment of the present invention provides a '161 KSP Inhibitor for administration to a patient having pathogenic cells in a state of '161 KSP Inhibitor-induced mitotic arrest, for increasing apoptosis of the cells, wherein the Inhibitor is administered at the maximum tolerated dose. In certain embodiments, the '161 KSP Inhibitor is Compound I. In certain embodiments, the '161 KSP Inhibitor is Compound 2. In certain embodiments, the '161 KSP Inhibitor is Compound 3. In certain embodiments, the '161 KSP Inhibitor is Compound 4. In certain embodiments, the '161 KSP Inhibitor is Compound 5. In certain embodiments, the '161 KSP Inhibitor is Compound 6. In certain embodiments, the pathogenic cells are cancer cells. In certain embodiments, the pathogenic cells are hematological tumor cells. In certain embodiments, the pathogenic cells are selected from lymphomas, leukemia and multiple myeloma cells. In certain embodiments, the pathogenic cells are solid tumor cells. In certain embodiments, the pathogenic cells are selected from tumor cells of the skin, breast, brain, cervical carcinoma, and testicular carcinoma cells. In certain embodiments, the solid tumor cells are selected from breast cancer, colorectal cancer, non-small cell lung cancer, pancreatic cancer, bladder cancer, salivary gland cancer (adenoid cystic), esophageal cancer, mesothelioma cancer, and mixed small cell lung cancer / non-small cell lung cancer.

### EXAMPLES

In order to illustrate the invention, the following Examples are included. However, it is to be understood that these Examples do not limit the invention and are only meant to support and suggest a method of practicing the invention.

### Example I

### Apoptosis washout

HT-29 cells, treated with either vehicle control (DMSO), or 10 nM Compound 4, were seeded in identical 96-well tissue culture plates. After 8 or 24 hours. Compound 4 was removed from HT-29 cells and replaced with fresh growth medium in order to determine whether apoptosis induction could be prevented. Caspase 3/7 activity was measured as reaction product luminescence at the indicated times using CaspaseGlo 3/7 reagent (Promega) and a luminometer. Values are reported as caspase 3/7 activity of Compound 4-treated cells divided by caspase 3/7 activity of DMSO-treated cells. The results are shown in Figure I.

### Example 2

### Apoptosis in HT-29 following continuous treatment with Compound 4

HT-29 cells, continuously treated with either vehicle control (DMSO), or 100 nM, 10 nM, 1 nM, or 0.1 nM Compound 4, were seeded in identical 96-well tissue culture plates. Caspase 3/7 activity was measured as luminescent reaction product at the indicated times using CaspaseGlo 3/7 reagent (Promega) and a luminometer. Values are reported as caspase 3/7 activity of Compound 4-treated cells divided by caspase 3/7 activity of DMSO-treated cells. Data include mean and standard deviation values from 4 independent experiments. The results are shown in Figure 2.

### Example 3

### Apoptosis in RPMI 8226

RPM1 8226 cells, treated with either vehicle control (DMSO), 10 nM Compound 4, or 10 nM vincristine were seeded in identical 96-well tissue culture plates. Caspase 3/7 activity was measured as luminescent reaction product at the indicated times using CaspaseGlo 3/7 reagent (Promega) and a luminometer. Values are reported as caspase 3/7 activity of drug-treated cells divided by caspase 3/7 activity of DMSO-treated cells. Data include mean and standard deviation values from 4 independent experiments. The results are shown in Figure 3.

### Example 4

### Duration of monopolar spindles and magnitude or apoptosis (HT-29 xenografts)

Female nude mice were implanted subcutaneously with 5 x 10⁶ HT-29 cells in 100 µL PBS. Ten days Inter, tumors were measured and mice randomized into groups of three with average tumor volume in each group of approximately 240 mm³. Compound 4 was dissolved in normal saline immediately before dosing. It was determined that 20 mg/kg was the MTD for Compound 4. Dose volume was 10 mL/kg. Dosing was vehicle alone on day 1; and Compound 4 at 20 mg/kg on day 1; and 20 mg/kg on days 1 and 3. At various time points after dosing (24, 48, 72, 96, 120 and 144 hours), mice were euthanized by CO₂ inhalation, and the tumors were harvested and immediately placed in formalin. The vehicle control group samples were collected 24 and 72 hours after dosing. The day I group samples were collected 24, 48, 72 and 96 hours after that dose. The day 1 and day 3 group samples were collected 72, 96, 120 and 144 hours after the first dose. Paraffin blocks of tumor tissue were prepared by standard procedures. Visualization of monopolar spindles was carried out by staining cut sections with mouse anti-human alpha tubulin primary antibody (clone B-7, Santa Cruz Biotechnology) followed by goat anti-mouse secondary antibody conjugated to Alexafluor 488 (Invitrogen). Nuclei were stained with Hoechst 33342 for cell counting. Spindle structures were manually counted in three 40X areas of each sample, using a fluorescent microscope. Apoptosis was quantitated by manual counting of TUNEL positive cells, also in three 40X areas of each sample (TUNEL staining using the In Situ Cell Death Detection Kit, AP from Roche). The results are shown in Figures 4 and 6.

### Example 5

### Duration of monopolar spindles and magnitude of apoptosis (HT-29 xenografts)

The methods of Example 5 are the same as Example 4, except that the dosing was vehicle alone on day 1; and Compound 4 at 8 mg/kg on day 1: and 8 mg/kg on days I and 3. The vehicle control group samples were collected 24 and 72 hours after dosing. The day I group samples were collected 24, 48, 72 and 96 hours after that dose. The days I and 3 group samples were collected 72, 96, 120 and 144 hours after the first dose. The results are shown in Figures 5 and 7.

### Example 6

### Mitotic block and apoptosis (HT-29)

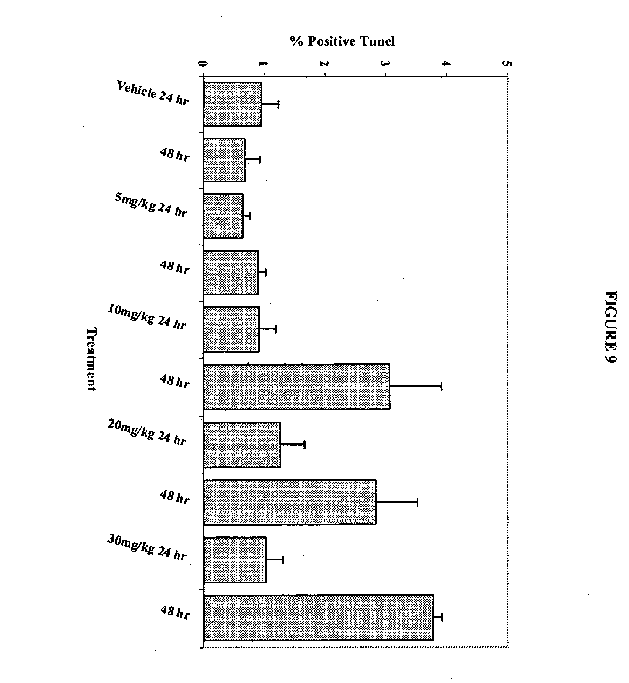

Female nude mice were implanted subcutaneously with 3 x 10⁶ HT-29 cells in 100 µL PBS. Fourteen days later, tumors were measured and mice randomized into groups of three with average tumor volume in each group of approximately 300 mm⁻³. Dosing was vehicle alone, and Compound 4 at 5, 10, 20 and 30 mg/kg. All samples were collected 24 and 48 hours after dosing. All other methods were as described for Example 4. The results are shown in Figures 8 and 9.

### Example 7

### Tumor Growth Inhibition on different dosing schedules (HT-29)

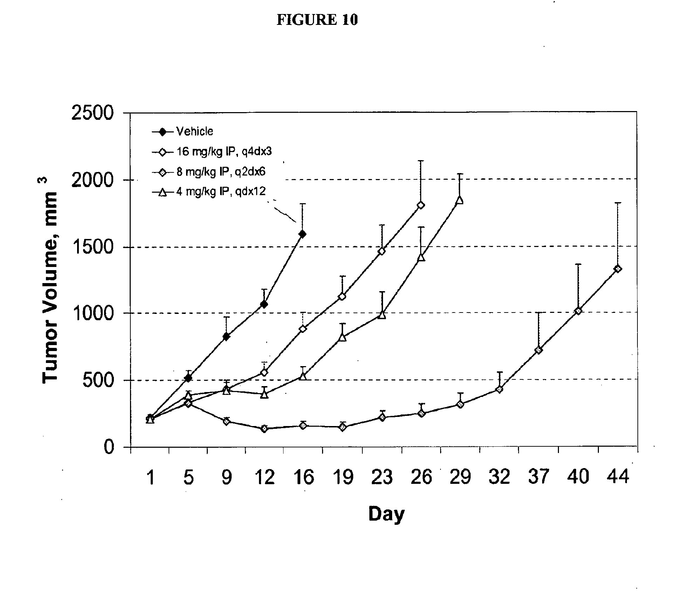

Female nude mice were implanted subcutaneously with 4 x 10⁶ HT-29 cells in 100 µL PBS. Thirteen days later, tumors were measured and mice randomized into groups of eight with average tumor volume in each group of approximately 210 mm³. Compound 4 was dissolved in normal saline immediately prior to dosing, and administered IP at a volume of 10 mL/kg for 12 days at doses of 4 mg/kg every day, 8 mg/kg every other day, and 16 mg/kg every fourth day. Animal weights and tumor volumes were measured (using electronic calipers) twice a week. Tumor volume was calculated using the formula: volume = (width² x length)/2. The results are shown in Figure 10.

### Example 8

### Tumor Growth Inhibition on different dosing schedules (HT-29)

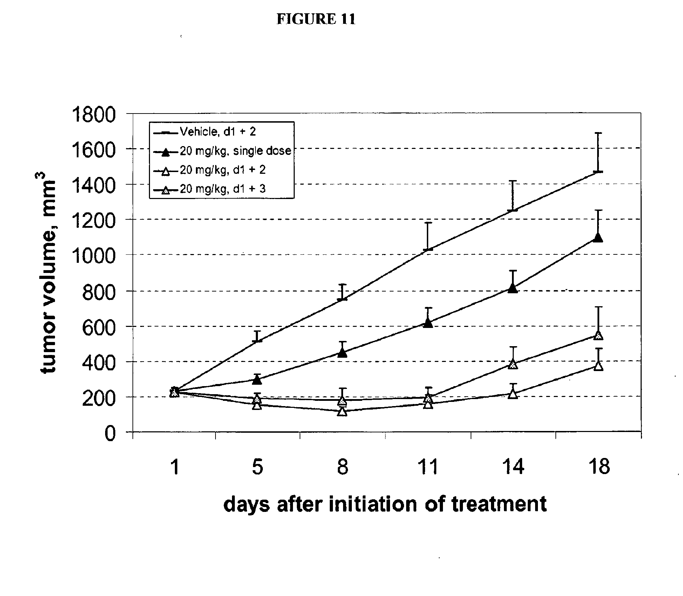

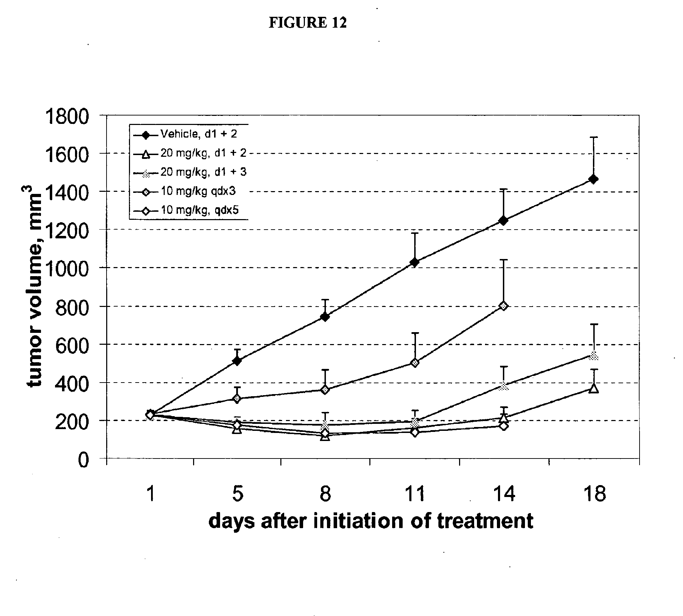

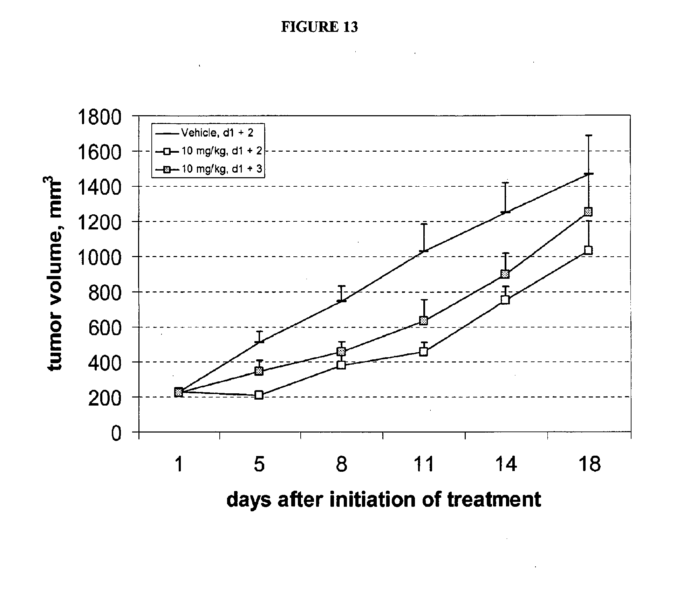

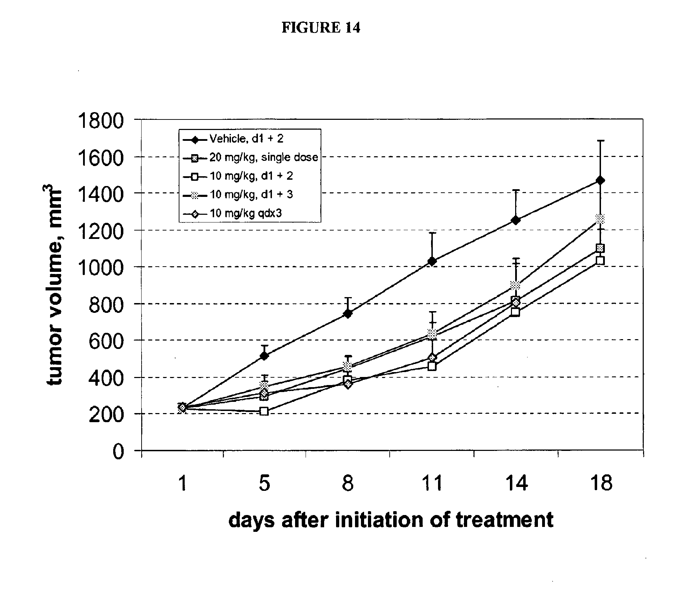

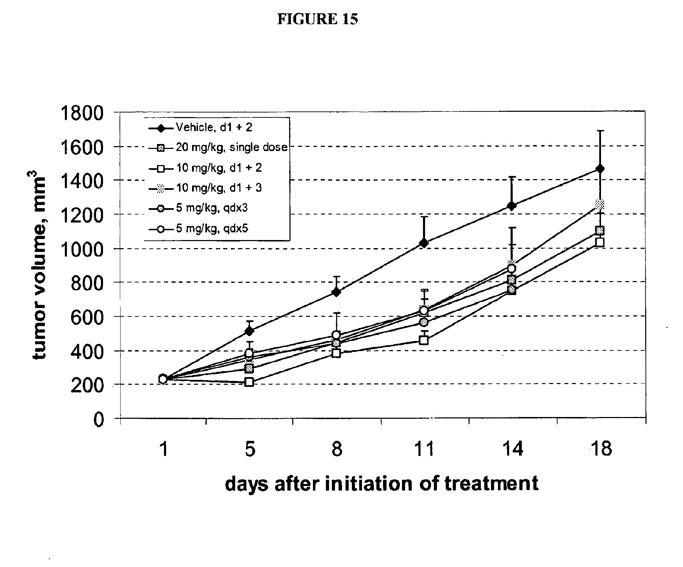

Female nude mice were implanted subcutaneously with 5 x 10⁶ HT-29 cells in 100 µL PBS. Eleven to fourteen days later, tumors were measured and mice randomized into groups of seven with average tumor volume in each group of approximately 230 mm³. Compound 4 was dissolved in normal saline immediately prior to dosing, and administered IP at a volume of 10 mL/kg. Dosing was vehicle alone on day 1 and day 2; and Compound 4 at 20 mg/kg on day 1 ; 20 mg/kg on days I and 2; 20 mg/kg on days 1 and 3; 5 mg/kg on days 1, 2 and 3; 10 mg/kg on days 1, 2 and 3; 10 mg/kg on days 1, 2, 3, 4 and 5; 10 mg/kg on days 1 and 2; and 10 mg/kg on days 1 and 3. Animal weights and tumor volumes were measured (using electronic calipers) twice a week. Tumor volume was calculated using the formula: volume = (width² x length)/2. Dosing at 10 mg/kg on days 1, 2, 3, 4 and 5 was not tolerated (greater than 20% weight loss and/or death in some of the mice). The results are shown in Figures 11-15.

### Example 9

### Tumor Growth Inhibition on different dosing schedules (RPMI 8226)

Female SCID-beige mice were implanted subcutaneously with I x 10⁷ RPMI 8226 cells in 100 µL PBS with 50% Matrigel. Twenty-five days later, tumors were measured and mice randomized into groups of seven with average tumor volume in each group of approximately 225 mm³. Compound 4 was dissolved in normal saline immediately prior to dosing, and administered IP at a volume of 10 mL/kg. Dosing was vehicle alone on day 1; and Compound 4 at 20 mg/kg on day 1; 10 mg/kg on days I and 2; 10 mg/kg on days I and 3; and 20 mg/kg on days 1, 5 and 9. Animal weights and tumor volumes were measured (using electronic calipers) twice a week. Tumor volume was calculated using the formula volume = (width² x length)/2. The results are shown in Figure 16.

### Example 10

### Duration of monopolar spindles, bipolar spindles and magnitude of apoptosis (RPMI 8226)

Female SCID-beige mice were implanted subcutaneously with 1 x 10⁷ RPMI 8226 cells in 100 µL PBS with 50% Matrigel. Thirty-one days later, tumors were measured and mice randomized into groups of three with average tumor volume in each group of approximately 210 mm³. Compound 4 was dissolved in normal saline immediately before dosing. Dose volume was 10 mL/kg. Dosing was vehicle alone on day 1; and Compound 4 at 10 mg/kg on day 1; 20 mg/kg on day 1; 10 mg/kg on days 1 and 2; and 10 mg/kg on days 1 and 3. At various times after dosing (24, 48, 72 and 96 hours), mice were euthanized by CO₂ inhalation, and tumors were harvested and immediately placed in formalin. The vehicle control group samples were collected 48 hours after dosing. The 10 mg/kg on day 1 samples were collected 24 and 48 hours after dosing. The 20 mg/kg on day 1 samples were collected 24, 48 and 72 hours after dosing. The 10 mg/kg on days 1 and 2 samples were collected 48 and 72 hours after the first dose. The 10 mg/kg on days 1 and 3 samples were collected 72 and 96 hours after the first dose. Paraffin blocks of tumor tissue were prepared by standard procedures. Visualization of monopolar spindles was carried out by staining cut sections with mouse anti-human alpha tubulin primary antibody (clone B-7, Santa Cruz Biotechnology), followed by goat anti-mouse secondary antibody conjugated to Alexafluor 488 (Invitrogen). Nuclei were stained with Hoechst 33342 for cell counting. Spindle structures were manually counted in three 40X areas of each sample, using a fluorescent microscope. Apoptosis was quantitated by manual counting of TUNEL positive cells, also in three 40X areas of each sample (TUNEL staining using the In Situ Cell Death Detection Kit, AP from Roche). The results are shown in Figures 17, 18 and 19.

### Example 11

### Determination of MTD in a Phase 1 Study

A total of 13 patients with various solid tumors and with a median age of 66 years (range 40-79 years old) were enrolled in a human phase 1 clinical trial (see "Phase 1 Safety and Phamacokinetic Study of ARRY-520 in Solid Tumors." http://clinicaltrials.gov/ct2/show/NCT00462358). The solid tumors treated were breast cancer (2), colorectal cancer (2), non-small cell lung cancer (2), pancreatic cancer (2), bladder cancer, salivary gland cancer (adenoid cystic), esophageal cancer, mesothelioma cancer, and a mixed small cell lung cancer / non-small cell lung cancer. Compound 4 was provided for administration as a lyophilized powder contained in a Type 1 clear glass vial for IV use. The dose levels administered were 1.25 and 1.6 mg/m²/day of Compound 4 on Days 1 and 2 every two weeks. The MTD was determined to be 1.25 mg/m²/day (cumulative dose per cycle of 2.5 mg/m²), with DLTs of Grade 3 hyponatremia, anorexia, AST increase and febrile neutropenia.

See also, "A Phase 1/2 Study of ARRY-520 in Patients With Relapsed or Refractory Multiple Myeloma." http://clinicaltrials.gov/et2/show/NCT00821249.

While the invention has been described in conjunction with the enumerated embodiments, it will be understood that they are not intended to limit the invention to those embodiments. On the contrary, the invention is intended to cover all alternatives, modifications and equivalents, which may be included within the scope of the present invention as defined by the claims. Thus, the foregoing description is considered as illustrative only of the principles of the invention.

The words "comprise," "comprising," "include," "including," and "includes" when used in this specification and in the following claims are intended to specify the presence of stated features, integers, components, or steps, but they do not preclude the presence or addition of one or more other features, integers, components, steps, or groups thereof.

## Claims

1. A '161 KSP Inhibitor for use in the treatment of pathogenic cells in a state of '161 KSP Inhibitor-induced mitotic arrest, for increasing apoptosis of the cells, wherein the '161 KSP Inhibitor is selected from 2-(3-aminopropyl)-5-(3-fluorophenyl)-N-(2-methoxyethyl)-N-methyl-2-phenyl-1,3,4-thiadiazole-3(2H)-carboxamide, 2-(3-aminopropyl)-5-(3-fluorophenyl)-N-methoxy-N-methyl-2-phenyl-1,3,4-thiadiazole-3(2H)-carboxamide, 2-(3-aminopropyl)-5-(2,5-difluorophenyl)-N-methoxy-N-methyl-2-phenyl-1,3,4-thiadiazole-3(2H)-carboxamide, (S)-2-(3-aminopropyl)-5-(2,5-difluorophenyl)-N-methoxy-N-methyl-2-phenyl-1,3,4-thiadiazole-3(2H)-carboxamide, (R)-2-(3-aminopropyl)-5-(2,5-difluorophenyl)-N-methoxy-N-methyl-2-phenyl-l ,3,4-thiadiazole-3(2H)-carboxamide, and 2-(3-aminopropyl)-5-(2,5-difluorophenyl)-N-hydroxy-N-methyl-2-phenyl-1,3,4-thiadiazole-3(2H)-carboxamide.

2. The Inhibitor for use according to Claim 1, which is the same as the inhibitor that has induced mitotic arrest.

3. The Inhibitor for use according to Claim 1 or 2, which is (S)-2-(3-aminopropyl)-5-(2,5-difluorophenyl)-N-methoxy-N-methyl-2-phenyl-1,3,4-thiadiazole-3(2H)-carboxamide.

4. The Inhibitor for use according to any of Claims 1 to 3, wherein the pathogenic cells are cancer cells.

5. The Inhibitor for use according to any of Claims 1 to 4, wherein the pathogenic cells are hematological tumor cells.

6. The Inhibitor for use according to any of Claims 1 to 5, wherein the pathogenic cells are selected from lymphomas, leukemia and multiple myeloma cells.

7. The Inhibitor for use according to any of Claims 1 to 4, wherein the pathogenic cells are solid tumor cells.

8. The Inhibitor for use according to any of Claims 1 to 4 or 7, wherein the pathogenic cells are selected from tumor cells of the skin, breast, brain, cervical carcinoma and testicular carcinoma cells.

9. The Inhibitor for use according to any of Claims 1 to 4 or 7, wherein the pathogenic cells are selected from breast cancer, colorectal cancer, non-small cell lung cancer, pancreatic cancer, bladder cancer, salivary gland cancer (adenoid cystic), esophageal cancer, mesothelioma cancer, and mixed small cell lung cancer / non-small cell lung cancer.

10. The Inhibitor for use according to any of Claims 1 to 9, wherein the Inhibitor is for administration at the maximum tolerated dose.

11. The Inhibitor for use according to any of Claims 3 to 10, wherein the maximum tolerated dose is 1.25 mg/m²/day.

12. The Inhibitor for use according to any of Claims 1 to 11, wherein the '161 Inhibitor for administration is administered 12 to 60 hours after the `161 Inhibitor that induced mitotic arrest.

13. The Inhibitor for use according to any of Claims 1 to 12, wherein the '161 Inhibitor for administration is administered 24 to 48 hours after the '161 Inhibitor that induced mitotic arrest.

14. The Inhibitor for use according to any of Claims 1 to 13, wherein the '161 Inhibitor is administered in a cycle from 11 to 24 days.

15. The Inhibitor for use according to any of Claims 1 to 14, wherein the '161 Inhibitor is administered in a cycle from 14 to 21 days.

## Patentansprüche

1. '161-KSP-Hemmer zur Verwendung bei der Behandlung von pathogenen Zellen in einem Zustand eines durch einen '161-KSP-Hemmer induzierten Mitosearrests zum Steigern der Apoptose der Zelle, wobei der '161-KSP-Hemmer aus 2-(3-Aminopropyl)-5-(3-fluorphenyl)-N-(2-methoxyethyl)-N-methyl-2-phenyl-1,3,4-thiadiazol-3(2H)-carboxamid, 2-(3-Aminopropyl)-5-(3-fluorphenyl)-N-methoxy-N-methyl-2-phenyl-1,3,4-thiadiazol-3(2H)-carboxamid, 2-(3-Aminopropyl)-5-(2,5-difluorphenyl)-N-methoxy-N-methyl-2-phenyl-1,3,4-thiadiazol-3(2H)-carboxamid, (S)-2-(3-Aminopropyl)-5-(2,5-difluorphenyl)-N-methoxy-N-methyl-2-phenyl-1,3,4-thiadiazol-3(2H)-carboxamid, (R)-2-(3-Aminopropyl)-5-(2,5-difluorphenyl)-N-methoxy-N-methyl-2-phenyl-1,3,4-thiadiazol-3(2H)-carboxamid und 2-(3-Aminopropyl)-5-(2,5-difluorphenyl)-N-hydroxy-N-methyl-2-phenyl-1,3,4-thiadiazol-3(2H)-carboxamid ausgewählt ist.

2. Hemmer zur Verwendung nach Anspruch 1, der mit dem Hemmer identisch ist, der den Mitosearrest induziert hat.

3. Hemmer zur Verwendung nach Anspruch 1 oder 2, bei dem es sich um (S)-2-(3-Aminopropyl)-5-(2,5-difluorphenyl)-N-methoxy-N-methyl-2-phenyl-1,3,4-thiadiazol-3(2H)-carboxamid handelt.

4. Hemmer zur Verwendung nach einem der Ansprüche 1 bis 3, wobei die pathogenen Zellen Krebszellen sind.

5. Hemmer zur Verwendung nach einem der Ansprüche 1 bis 4, wobei die pathogenen Zellen hämatologische Tumorzellen sind.

6. Hemmer zur Verwendung nach einem der Ansprüche 1 bis 5, wobei die pathogenen Zellen aus Lymphomen, Leukämiezellen und Zellen eines multiplen Myeloms ausgewählt sind.

7. Hemmer zur Verwendung nach einem der Ansprüche 1 bis 4, wobei die pathogenen Zellen Zellen eines soliden Tumors sind.

8. Hemmer zur Verwendung nach einem der Ansprüche 1 bis 4 oder 7, wobei die pathogenen Zellen aus Tumorzellen der Haut, der Brust, des Gehirns, Zervixkarzinom- und Hodenkarzinomzellen ausgewählt sind.

9. Hemmer zur Verwendung nach einem der Ansprüche 1 bis 4 oder 7, wobei die pathogenen Zellen aus Brustkrebs, kolorektalem Karzinom, nichtkleinzelligem Lungenkrebs, Bauchspeicheldrüsenkrebs, Blasenkrebs, Speicheldrüsenkrebs (adenoidzystisches Karzinom), Speiseröhrenkrebs, Mesotheliom-Karzinom und gemischtem kleinzelligem Lungenkrebs/nichtkleinzelligem Lungenkrebs ausgewählt sind.

10. Hemmer zur Verwendung nach einem der Ansprüche 1 bis 9, wobei der Hemmer zur Verabreichung in der maximal tolerierten Dosis ist.

11. Hemmer zur Verwendung nach einem der Ansprüche 3 bis 10, wobei die maximal tolerierte Dosis 1,25 mg/m²/Tag ist.

12. Hemmer zur Verwendung nach einem der Ansprüche 1 bis 11, wobei der '161-Hemmer zur Verabreichung 12 bis 60 Stunden nach dem '161-Hemmer, der den Mitosearrest induziert hat, verabreicht wird.

13. Hemmer zur Verwendung nach einem der Ansprüche 1 bis 12, wobei der '161-Hemmer zur Verabreichung 24 bis 48 Stunden nach dem '161-Hemmer, der den Mitosearrest induziert hat, verabreicht wird.

14. Hemmer zur Verwendung nach einem der Ansprüche 1 bis 13, wobei der '161-Hemmer in einem Zyklus von 11 bis 24 Tagen verabreicht wird.

15. Hemmer zur Verwendung nach einem der Ansprüche 1 bis 14, wobei der '161-Hemmer in einem Zyklus von 14 bis 21 Tagen verabreicht wird.

## Revendications

1. Inhibiteur'161 de la KSP pour son utilisation dans le traitement de cellules pathogènes dans un état d'arrêt mitotique induit par l'inhibiteur '161 de la KSP, à des fins d'augmentation de l'apoptose des cellules, dans lequel l'inhibiteur '161 de la KSP est choisi parmi le 2-(3-aminopropyl)-5-(3-fluorophényl)-N-(2-méthoxyéthyl)-N-méthyl-2-phényl-1,3,4-thiadiazole-3(2H)-carboxamide, le 2-(3-aminopropyl)5-(3-fluorophényl)-N-méthoxy-N-méthyl-2-phényl-1,3,4-thiadiazole-3(2H)-carboxamide, le 2(3-aminopropyl)-5-(2,5-difluorophényl)-N-méthoxy-N-méthyl-2-phényl-1,3,4-thiadiazole3(2H)-carboxamide, le (S)-2-(3-aminopropyl)-5-(2,5-difluorophényl)-N-méthoxy-N-méthyl-2-phényl-1,3,4-thiadiazole-3(2H)-carboxamide, le (R)-2-(3-aminopropyl)-5-(2,5-difluorophényl)-N-méthoxy-N-méthyl-2-phényl-1,3,4-thiadiazole-3(2H)-carboxamide et le 2-(3-aminopropyl)-5-(2,5-difluorophényl)-N-hydroxy-N-méthyl-2-phényl-1,3,4-thiadiazole-3(2H)-carboxamide.

2. Inhibiteur pour son utilisation selon la revendication 1, qui est identique à l'inhibiteur qui a induit l'arrêt mitotique.

3. Inhibiteur pour son utilisation selon la revendication 1 ou 2, à savoir le (S)-2-(3-aminopropyl)-5-(2,5-difluorophényl)-N-méthoxy-N-méthyl-2-phényl-1,3,4-thiadiazole-3(2H)-carboxamide.

4. Inhibiteur pour son utilisation selon l'une quelconque des revendications 1 à 3, dans lequel les cellules pathogènes sont des cellules cancéreuses.

5. Inhibiteur pour son utilisation selon l'une quelconque des revendications 1 à 4, dans lequel les cellules pathogènes sont des cellules tumorales hématologiques.

6. Inhibiteur pour son utilisation selon l'une quelconque des revendications 1 à 5, dans lequel les cellules pathogènes sont choisies parmi des cellules de lymphome, des cellules de leucémie et des cellules de myélome multiple.

7. Inhibiteur pour son utilisation selon l'une quelconque des revendications 1 à 6, dans lequel les cellules pathogènes sont des cellules tumorales solides.

8. Inhibiteur pour son utilisation selon l'une quelconque des revendications 1 à 4 ou 7, dans lequel les cellules pathogènes sont choisies parmi des cellules tumorales de carcinome de la peau, du sein, du cerveau, du col de l'utérus et des cellules de carcinome testiculaire.

9. Inhibiteur pour son utilisation selon l'une quelconque des revendications 1 à 4 ou 7, dans lequel les cellules pathogènes sont choisies parmi le cancer du sein, le cancer colorectal, le cancer du poumon non à petites cellules, le cancer pancréatique, le cancer de la vessie, le cancer des glandes salivaires (carcinome adénoïde kystique), le cancer de l'oesophage, le mésothéliome et un cancer mixte du poumon à petites cellules/du poumon non à petites cellules.

10. Inhibiteur pour son utilisation selon l'une quelconque des revendications 1 à 9, dans lequel l'inhibiteur est destiné à une administration à la dose maximale tolérée.

11. Inhibiteur pour son utilisation selon l'une quelconque des revendications 3 à 10, dans lequel la dose maximale tolérée s'élève à 1,25 mg/m²/jour.

12. Inhibiteur pour son utilisation selon l'une quelconque des revendications 1 à 11, dans lequel l'inhibiteur '161 pour l'administration est administré 12 à 60 heures après l'inhibiteur'161 qui a induit l'arrêt mitotique.

13. Inhibiteur pour son utilisation selon l'une quelconque des revendications 1 à 12, dans lequel l'inhibiteur '161 pour l'administration est administré 24 à 48 heures après l'inhibiteur'161 qui a induit l'arrêt mitotique.

14. Inhibiteur pour son utilisation selon l'une quelconque des revendications 1 à 13, dans lequel l'inhibiteur '161 est administré dans un cycle de 11 à 24 jours.

15. Inhibiteur pour son utilisation selon l'une quelconque des revendications 1 à 14, dans lequel l'inhibiteur '161 est administré dans un cycle de 14 à 21 jours.
